**Europäisches Patentamt**

**European Patent Office**

**Office européen des brevets**

(11) Veröffentlichungsnummer: **0 208 988**
**B1**

(12) **EUROPÄISCHE PATENTSCHRIFT**

(45) Veröffentlichungstag der Patentschrift:
18.10.89

(51) Int. Cl.⁴: **B01J 23/02,** C07C 37/16,
B01J 27/20

(21) Anmeldenummer: **86108981.1**

(22) Anmeldetag: **02.07.86**

(54) Verfahren zur Gasphasenalkylierung von Phenolen und hierzu geeigneter Katalysator.

(30) Priorität: 08.07.85 DE 3524331

(43) Veröffentlichungstag der Anmeldung:
21.01.87 Patentblatt 87/4

(45) Bekanntmachung des Hinweises auf die Patenterteilung:
18.10.89 Patentblatt 89/42

(84) Benannte Vertragsstaaten:
BE DE FR GB IT NL

(56) Entgegenhaltungen:
EP-A- 0 038 377
EP-A- 0 129 065
EP-A- 0 150 311
FR-A- 1 484 240
NL-A- 251 724
US-A- 3 968 172

Die Akte enthält technische Angaben, die nach dem Eingang der Anmeldung eingereicht wurden und die nicht in dieser Patentschrift enthalten sind.

(73) Patentinhaber: BASF Aktiengesellschaft,
Carl-Bosch-Strasse 38, D-6700 Ludwigshafen(DE)

(72) Erfinder: Lenz, Hans-Heinrich, Dr., Geminiweg 2,
D-4950 Minden(DE)
Erfinder: Roske, Eckhard, Hillensheimer Strasse 4,
D-6700 Ludwigshafen(DE)
Erfinder: Baer, Karl, dr., Kastanienweg 1,
D-6940 Weinheim(DE)

ACTORUM AG

**Beschreibung**

Die Erfindung betrifft ein Verfahren zur Gasphasenalkylierung von Phenolen und einen hierzu geeigneten Katalysator. Die Gasphasenalkylierung erfolgt durch Umsetzung von Phenolen, die in einer und/oder zwei ortho-Stellungen Wasserstoff tragen, mit Alkoholen in Gegenwart des genannten Katalysators unter Bildung der entsprechenden, in ortho-Stellung alkyl-substituierten Phenole.

In der Literatur sind eine ganze Reihe von Systemen beschrieben worden, die diese Alkylierung katalysieren. Die bekannten Katalysatorsysteme weisen aber stets Nachteile im Hinblick auf mindestens eines der folgenden Merkmale auf:

Aktivität

ortho-Selektivität (d.h. Ausbeute für die Alkylierung in ortho-Stellung bezüglich umgesetztem Phenol)

Alkanol-Selektivität

Standzeit

maximale Dicke des Kontaktrohres

mechanische Stabilität

Notwendigkeit von Zusätzen zum Kontakt oder Zulauf (Inertmaterial).

So ist in der US-A 3 446 856 ein Katalysator auf der Basis von Magnesiumoxid beschrieben, der eine Standzeit von lediglich 75 bis 100 Stunden hat und dessen Methanolselektivität bei nur 40 bis 50% liegt. Der in der US-A 2 448 942 beschriebene Katalysator auf der Basis von Aluminiumoxid ist insofern nachteilig, als er eine geringe ortho-Selektivität aufweist. Die britische Patentschrift 1 034 500 beschreibt einen Magnesiumoxid-Katalysator, der Reaktionstemperaturen von über 500°C benötigt, also wenig aktiv ist. Der Magnesiumoxid-Katalysator gemäß DE-B 1 263 010 ist insofern nachteilig, als er sich nicht in eine mechanisch stabile Form bringen läßt. Toxikologisch bedenklich ist ein Katalysator auf der Basis Fe/Si/Cr, so wie er in der DE-A 2 428 056 beschrieben ist. Das Katalysatorsystem gemäß DE-A 3 228 713 auf Fe/Si/Mg ist in einem Ofen einsetzbar, der in mehreren Zonen beheizbar sein muß, um die Reaktion isotherm fahren zu können. Die DE-A 3 149 022 beschreibt ein Katalysatorsystem aus Fe/Cr/Ce, bei dem durch spezielle Maßnahmen die Temperaturdifferenz klein (unter 5°C) gehalten werden muß, um eine Methanolzersetzung zu vermeiden. Diese Maßnahmen sind enge Rohrquerschnitte (< 50 mm), Verdünnung des Katalysators und Verdünnung des Eduktes, jeweils mit Inertmaterial. Das Katalysatorsystem der DE-C 3 012 357 ist ein kompliziertes Vier-Komponenten-System aus Fe/Si/Cr/Erdalkali. Nachteilig ist der Umstand, daß man, bezogen auf Phenol, einen großen Methanolüberschuß einsetzen muß. Außerdem erfolgt Zersetzung des Methanols. Die DE-A 2 853 452 beschreibt ein Katalysatorsystem aus Mn/Si/Erdalkali, dem ein geeignetes Inertgas, wie Stickstoff oder Kohlendioxid zugesetzt werden muß, um die Reaktion glatt ablaufen zu lassen. Das wiederum erschwert die Aufarbeitung. Die EP-B 19 476 beschreibt ein Katalysatorsystem aus Eisen und Gallium, bei dem nachteilig ist, daß die Methanolselektivität kleiner als 70% ist. Eine geringe ortho-Selektivität weist das Katalysatorsystem aus Mg/Ti/Sulfat gemäß US-A 4 283 574 auf. Die DE-A 2 716 035 betrifft einen Katalysator aus Kupfer und Chrom, bei dem nachteilig ist, daß er bei niedriger Temperatur (250 bis 300°C) eine geringe Aktivität aufweist. Wenn man die Temperatur steigert, um den Umsatz zu erhöhen (beispielsweise auf 325°C) erfolgt eine beinahe vollständige Methanolzersetzung. Der in der GB-B 2 089 343 beschriebene Katalysator aus Fe/Cu und Chrom ist insofern nachteilig, als bereits nach kurzer Betriebsdauer Aktivität und Selektivität verloren gehen.

In der US-A 3 968 172 wird ein Katalysator auf Magnesiumoxidbasis beschrieben, wobei bis zu 10 Gew.-% des Magnesiumoxids im Katalysator durch andere Metallverbindungen, z.B. Alkalimetallhydroxide, ersetzt sein können. Die Verwendung von unter anderem Graphit als Formhilfsmittel bei der Katalysatorherstellung wird in dieser Patentschrift bedacht, ist jedoch nicht weiter belegt. Die in dieser Patentschrift beschriebenen Katalysatoren katalysieren die Gasphasenalkylierung von Phenolen in ortho- und para-Stellung – eine hohe ortho-Selektivität bei der Alkylierung kann mit diesen Katalysatoren nicht erzielt werden. Ein weiterer Nachteil dieser Katalysatoren ist ihre relativ geringe Standzeit.

Der Erfindung liegt die Aufgabe zugrunde, einen Katalysator für die Gasphasenalkylierung von Phenolen auf der Basis von Magnesiumoxid bereitzustellen, der die genannten Nachteile nicht aufweist und insbesondere bei hoher Ausbeute und Selektivität ohne Aktivitätsverlust eine lange Standzeit besitzt. Es soll auch ein Verfahren zur Gasphasenalkylierung von Phenolen unter Verwendung eines derartigen Katalysators geschaffen werden.

Dementsprechend wurde ein Katalysator für die Gasphasenalkylierung von Phenolen auf der Basis von Magnesiumoxid mit einem Alkaligehalt, ausgedrückt als Alkalimetalloxid und bezogen auf das Gesamtgewicht des Katalysators, von 0,05 bis 0,4 Gew.-% gefunden, der dadurch gekennzeichnet ist, daß er mit 1 bis 8 Gew.-% Graphit, bezogen auf das Gesamtgewicht des Katalysators, mechanisch befestigt, geformt und bei 550 bis 700°C getempert worden ist.

Des weiteren wurde ein Verfahren zur Gasphasenalkylierung von Phenolen gefunden, das dadurch gekennzeichnet ist, daß man einen Katalysator nach einem der Ansprüche 1 bis 6 verwendet.

Beim Verfahren zur Gasphasenalkylierung werden solche Phenole eingesetzt, die in einer oder beiden ortho-Stellungen zur Hydroxygruppe Wasserstoff tragen und mit Alkoholen unter Verwendung eines Katalysators auf Magnesiumoxidbasis umgesetzt werden. Das Verfahren ist dadurch gekennzeichnet, daß der Katalysator einen Alkalioxidgehalt von 0,05 bis 0,4 Gew.-% aufweist und mit 1 bis 8 Gew.-% Graphit, jeweils bezogen auf das Gesamtgewicht des Katalysators, mechanisch befestigt, geformt und getempert ist.

Nach einer bevorzugten Ausführungsform des Katalysators beträgt der Alkalioxidgehalt 0,05 bis 0,2 Gew.-%, insbesondere 0,1 Gew.-%, jeweils bezogen auf das Gesamtgewicht des Katalysators.

Bevorzugte Alkalikomponente sind Natriumionen. Aber auch andere Alkaliionen können eingesetzt werden, beispielsweise Lithium, Kalium, Rubidium und Cäsium.

Der Katalysator ist mit 1 bis 8 Gew.-% Graphit mechanisch befestigt und geformt. Bevorzugt ist ein Gehalt von 1,5 bis 3 Gew.-%, insbesondere von ungefähr 2 Gew.-%, Graphit, jeweils bezogen auf das Gesamtgewicht des Katalystors.

Der Katalysator kann sämtliche üblichen Formen aufweisen, beispielsweise kann er in Form von Ringen, Pellets, Kugeln oder Kegeln vorliegen.

Das Formen des erfindungsgemäßen Katalysators erfolgt bevorzugt durch Tablettieren. Anschließend wird bei 550 bis 700°C, bevorzugt bei 600 bis 650°C während eines ausreichenden Zeitraums, vorzugsweise 1 Stunde, getempert.

Der erfindungsgemäße Katalysator weist überraschend die eingangs in Verbindung mit dem Stand der Technik geschilderten Nachteile nicht auf. Die Umsätze sind nahezu quantitativ, wobei eine hohe ortho-Selektivität gewährleistet ist. Die Standzeit des Katalysators ist bemerkenswert lang. Auch nach einer Betriebszeit von 2000 Stunden treten kaum merkliche Verminderungen der Aktivität auf, wobei die Selektivität erhalten bleibt.

Abweichungen in der Zusammensetzung führen zu einer Verschlechterung der Kontakteigenschaften. Niedrigere Alkaligehalte führen zu einer Verminderung der Standzeit, während höhere Alkaligehalte zur Verminderung der Aktivität und der Methanolselektivität führen.

Niedrigere Graphitgehalte als die angegebenen erschweren die Tablettierbarkeit. Höhere Graphitgehalte vermindern die Standzeit, die Aktivität und die ortho-Selektivität. Andere organische Tablettierhilfsmittel vermindern ebenfalls Standzeit und Aktivität. Anorganische Tablettierhilfsmittel sind nicht geeignet.

Stränge sind mechanisch nicht stabil. Die Temperung bei niedrigen Temperaturen führt zu niedriger ortho-Selektivität, bei höheren Temperaturen als den angegebenen, zu einer Desaktivierung.

Beim erfindungsgemäßen Verfahren zur Gasphasenalkylierung wird das Ausgangsgemisch aus Phenol, Alkanol und gegebenenfalls Wasser in der üblichen Weise verdampft und dem Reaktor zugeführt. Der Katalysator ist zweckmäßig in einem Rohr angeordnet. Die Temperatur wird durch ein Heizmedium, beispielsweise ein Salzbad, auf 400 bis 500°C, vorzugsweise 420 bis 480°C, eingestellt.

Beim erfindungsgemäßen Verfahren zur Gasphasenalkylierung werden ohne jeglichen Verdünnungseffekt oder zusätzliche Heizzonen starke Temperaturerhöhungen, sogenannte "hot spots" vermieden. Hierdurch ist beispielsweise bei der Umsetzung von Phenol und Methanol die Methanolselektivität mit 85 bis 95% höher als bei den bekannten Verfahren. Da keine "hot spots" auftreten, können Kontaktrohre mit einem Durchmesser von > 50 mm eingesetzt werden.

Beim erfindungsgemäßen Verfahren ist der Phenolumsatz nahezu quantitativ. Die ortho-Selektivität liegt bei > 95%. Nach einer Betriebszeit von wenigen Tagen liegt sie sogar bei 97 bis 98%.

Sollte die Aktivität des erfindungsgemäßen Katalysators nach sehr langer Betriebszeit absinken, ist ein Regenerieren im Reaktionsrohr mit Stickstoff/Sauerstoff-Gemischen möglich.

Im erfindungsgemäßen Verfahren zur Gasphasenalkylierung lassen sich Phenole einsetzen, die in einer oder beiden ortho-Stellungen zur Hydroxygruppe Wasserstoff tragen. Man kann unsubstituiertes Phenol oder substituierte Phenole, unsubstituiertes Naphthol, beispielsweise α-Naphthol, oder substituierte Naphthole, einsetzen. Besonders bevorzugt ist unsubstituiertes Phenol.

Als Alkohole lassen sich primäre, sekundäre oder tertiäre aliphatische, gesättigte oder ungesättigte Alkohole einsetzen. Beispiele sind Methanol, Ethanol, n-Propanol, Isopropanol, n-Butanol, Isobutanol, tert.-Butanol, sec.-Butanol, Pentanole, Hexanole, Octanole und Decanole. Besonders bevorzugt sind Methanol, Ethanol und Isopropanol.

Nach dem erfindungsgemäßen Verfahren lassen sich beispielsweise unter Verwendung von Methanol die folgenden Phenole zu den entsprechenden Wertprodukten umsetzen:

Phenol zu o-Kresol und/oder 2,6-Dimethylphenol, o-Kresol zu 2,6-Dimethylphenol, m-Kresol oder 2,3-Dimethylphenol zu 2,3,6-Trimethylphenol, p-Kresol zu 2,4,6-Trimethylphenol, 3,5-Dimethylphenol zu 2,3,5,6-Tetramethylphenol, 3,4-Dimethylphenol zu 2,4,5-Trimethylphenol, 2,3,4-Trimethylphenol zu 2,3,4,6-Tetramethylphenol, 2,5-Dimethylphenol zu 2,3,6-Trimethylphenol, 4-tert.-Butylphenol zu 2,6-Dimethyl-4-tert.-butylphenol.

Die Reaktion von α-Naphthol mit Methanol kann beispielsweise 2-Methyl-α-naphthol ergeben.

Setzt man Phenol mit Ethanol um, so lassen sich beispielsweise 2-Ethylphenol und 2,6-Diethylphenol herstellen.

Setzt man Phenol mit Isopropanol um, so kann man beispielsweise 2-Isopropylphenol herstellen.

Soll aus einem Phenol das Dialkylderivat hergestellt werden, kann das primär entstehende Monoalkylderivat in den Reaktor zurückgeführt werden.

Der erfindungsgemäße Katalysator weist einen Alkaligehalt von 0,05 bis 0,4 Gew.-%, berechnet als Alkalimetalloxid und bezogen auf das Gesamtgewicht des Katalysators, auf.

Das nachfolgende Beispiel und die Vergleichsbeispiele 1 bis 3 dienen zur weiteren Erläuterung der Erfindung.

Beispiel

Ein beheizbarer Festbettreaktor wird mit 450 ml eines Magnesiumoxids mit einem $Na_2O$-Gehalt von 0,1%, das mit einem Zusatz von 2% Graphit tablettiert (5 mm-Körnung) und 1 h bei 650°C getempert wurde, gefüllt. Bei einer Kontakttemperatur von 465°C werden 442,5 g/h eines Gemisches aus Phenol, o-Kresol, Methanol und Wasser im molaren Verhältnis 1:1:10:4 dampfförmig bei Normaldruck über eine Kontaktstrecke von 60 cm Länge geleitet. Das austretende Reaktionsgemisch wird kondensiert, der gasförmige Anteil, der hauptsächlich aus Wasserstoff, Kohlenmonoxid, Kohlendioxid und Methan besteht, abgeleitet. Nach der destillativen Abtrennung des Methanols und des Wassers verbleibt ein Austrag der folgenden Zusammensetzung.

1,6% Phenol
31,1% o-Kresol
66,9% 2,6-Dimethylphenol.

ortho-Selektivität und Methanolselektivität betragen 98% bzw. 89%. Nach 42 Tagen Betriebsdauer zeigte der Kontakt nicht den geringsten Aktivitätsverlust.

Vergleichsbeispiel 1

In einem Festbettreaktor wurden 50 ml eines mit 2% Graphitzusatz tablettierten (2 bis 3 mm Körnung) Magnesiumoxides mit einem Na₂O-Gehalt von 0,1%, das 1 h bei 750°C getempert wurde, eingefüllt. Über die Kontaktstrecke von 60 cm Länge wurden dampfförmig 59,4 g/h eines Gemisches aus Phenol, o-Kresol, Methanol und Wasser im molaren Verhältnis 1:1:10:4 geleitet. Die Temperatur in der Kontaktzone mußte wegen geringerer Kontaktaktivität auf 520°C eingestellt werden. Nach Aufarbeitung des kondensierten Austrages erhielt man

13,8% Phenol
68,8% o-Kresol
15,5% 2,6-Dimethylphenol.

ortho-Selektivität und Methanolselektivität lagen bei 96% bzw. 41%. Der Kontakt zeigte schon mit Beginn der 15tägigen Betriebsdauer eine schleichende Aktivitätsabnahme.

Vergleichsbeispiel 2

Es wurde ein Magnesiumoxid verwendet, das mit 1,0% Na₂O dotiert war und mit 2% Graphitzusatz tablettiert, 1 h bei 650°C getempert worden war. Die Reaktionsbedingungen entsprachen dem Vergleichsbeispiel 1. Im aufgearbeiteten Kondensat wurden gefunden:

26,9% Phenol
59,6% o-Kresol
7,7% 2,6-Dimethylphenol.

ortho-Selektivität und Methanolselektivität betrugen 90,3% bzw. 37%. Der Kontakt konnte das von vornherein niedrige Aktivitätsniveau nicht über eine Dauer von 5 Betriebstagen halten.

Vergleichsbeispiel 3

Es wurde ein Magnesiumoxid mit Na₂O-Gehalt = 0,1%, tablettiert mit 10% Graphitzusatz und 1 h bei 650°C getempert, wie in Vergleichsbeispiel 1, aber bei 490°C, geprüft. Im aufgearbeiteten, kondensierten Austrag wurden gefunden:

5,2% Phenol
55,8% o-Kresol
36,5% 2,6-Dimethylphenol.

ortho-Selektivität und Methanolselektivität lagen bei 96% bzw. 42,2%. Der Kontakt zeigte innerhalb von 30 Tagen Betriebsdauer eine erhebliche Aktivitätsabnahme.

**Patentansprüche**

1. Katalysator für die Gasphasenalkylierung von Phenolen auf der Basis von Magnesiumoxid mit einem Alkaligehalt, ausgedrückt als Alkalimetalloxid und bezogen auf das Gesamtgewicht des Katalysators, von 0,05 bis 0,4 Gew.-%, dadurch gekennzeichnet, daß er mit 1 bis 8 Gew.-% Graphit, bezogen auf das Gesamtgewicht des Katalisators, mechanisch befestigt, geformt und bei 550 bis 700°C getempert worden ist.

2. Katalysator nach Anspruch 1, dadurch gekennzeichnet, daß der Alkalioxidgehalt 0,05 bis 0,2 Gew.-% beträgt.

3. Katalysator nach einem der Ansprüche 1 oder 2, dadurch gekennzeichnet, daß er als Alkalikomponente Natriumionen enthält.

4. Katalysator nach einem der Ansprüche 1 bis 3, dadurch gekennzeichnet, daß er 1,5 bis 3 Gew.-% Graphit aufweist.

5. Katalysator nach einem der Ansprüche 1 bis 4, dadurch gekennzeichnet, daß er durch Tempern bei 550 bis 700°C erhältlich ist.

6. Katalysator nach einem der Ansprüche 1 bis 5, dadurch gekennzeichnet, daß er durch Tablettieren geformt ist.

7. Verfahren zur Gasphasenalkylierung von Phenolen, die in einer oder beiden ortho-Stellungen zur Hydroxygruppe Wasserstoff tragen, mit Alkoholen unter Verwendung eines Katalysators auf Magnesiumoxidbasis mit einem Alkaligehalt, ausgedrückt als Alkalimetalloixid und bezogen I auf das Gesamtgewicht des Katalysators, von 0,05 bis 0,4 Gew.-%, dadurch gekennzeichnet, daß man einen Katalysator nach einem der Ansprüche 1 bis 6 verwendet.

**Claims**

1. A catalyst for the gas-phase alkylation of phenols which is based on magnesium oxide and has an alkali metal content, expressed as alkali metal oxide and based on the total weight of the catalyst, of from 0.05 to 0.4% by weight, wherein said catalyst has been mechanically stabilized with from 1 to 8% by weight, based on the total weight of the catalyst, of graphite, molded and heated at from 550 to 700°C.

2. A catalyst as claimed in claim 1, wherein the alkali metal oxide content is from 0.05 to 0.2% by weight.

3. A catalyst as claimed in either of claims 1 and 2, which contains sodium ions as the alkali metal component.

4. A catalyst as claimed in any of claims 1 to 3, which contains from 1.5 to 3% by weight of graphite.

5. A catalyst as claimed in any of claims 1 to 4, which is obtainable by heating at from 550 to 700°C.

6. A catalyst as claimed in any of claims 1 to 5, which is molded by pelletizing.

7. A process for the gas-phase alkylation of phenols, which carry hydrogen in one or both positions ortho to the hydroxyl group, with alcohols, using a catalyst which is based on magnesium oxide and has an alkali metal content, expressed as alkali metal oxide and based on the total weight of the catalyst, of from 0.05 to 0.4% by weight, which comprises using a catalyst as claimed in any of claims 1 to 6.

**Revendications**

1. Catalyseur pour l'alkylation en phase gazeuse de phénols, à base d'oxyde de magnésium avec une teneur en métal alcalin, exprimée en oxyde de métal alcalin et par rapport au poids total du catalyseur, de 0,05 à 0.4% en poids caractérisé en ce qu'il a été renforcé mécaniquement par 1 à 8% en poids de graphite, par rapport au poids total du catalyseur, façonné et recuit à une température de 550 à 700°C.

2. Catalyseur selon la revendication 1, caractérisé en ce que la teneur en oxyde alcalin est de 0,05 à 0.2% en poids.

3. Catalyseur selon l'une ou l'autre des revendications 1 et 3, caractérisé en ce que, comme composant alcalin, il contient des ions sodium.

4. Catalyseur selon l'une des revendications 1 à 3, caractérisé en ce qu'il contient de 1,5 à 3% en poids de graphite.

5. Catalyseur selon l'une des revendications 1 à 4, caractérisé en ce qu'il est préparé par recuit à 550–700°C.

6. Catalyseur selon l'une des revendications 1 à 5, caractérisé en ce qu'il est façonné par formation de pastilles.

7. Procédé pour l'alkylation en phase gazeuse de phénols, qui portent de l'hydrogène sur l'une ou les deux positions ortho par rapport au groupement hydroxy, avec des alcools en présence d'un catalyseur à base d'oxyde de magnésium ayant une teneur en métal alcalin, exprimée en oxyde de métal alcalin et par rapport au poids total du catalyseur, de 0,05 à 0,4% en poids, caractérisé en ce qu'on utilise un catalyseur selon l'une quelconque des revendications 1 à 6.